# EUROPEAN PATENT SPECIFICATION

(11) **EP 4 424 038 B1**
(45) Date of publication and mention of the grant of the patent: **09.12.2026**
(21) Application number: 23808647.4
(22) Date of filing: 09.02.2023
(51) Int. Cl.: A01J 9/04, G06Q 10/08, G01N 33/04, G06Q 50/02, H04W 4/38

(54) **IN-TANK RAW MILK MONITORING SYSTEM AND METHOD**
SYSTEM UND VERFAHREN ZUR ÜBERWACHUNG VON ROHMILCH IM TANK
SYSTÈME ET PROCÉDÉ DE SURVEILLANCE DE LAIT CRU DANS UN RÉSERVOIR

(30) Priority: 13.01.2023 TR 202300448
(43) Date of publication of application: 04.09.2024
(73) Proprietor: Rawbox Teknoloji A.S, Kocaeli (TR)
(72) Inventor: CUBUKCU, Nüzhet Emir, Kocaeli (TR)
(74) Representative: Valet Patent Services Limited
(86) International application number: PCT/TR2023/050119
(87) International publication number: WO 2024/151231

(56) References cited:
- EP-A1- 3 854 205
- WO-A1-2021/001378
- US-A1- 2021 176 950
- US-A1- 2022 268 620
- KARRAS ARISTEIDIS, KARRAS CHRISTOS, DRAKOPOULOS GEORGIOS, TSOLIS DIMITRIOS, MYLONAS PHIVOS, SIOUTAS SPYROS: "SAF: The Design Of A P2P-IoT LoRa System For Smart Agriculture Supply Chains", 18TH INTERNATIONAL CONFERENCE ON ARTIFICIAL INTELLIGENCE APPLICATIONS AND INNOVATIONS (AIAI 2022), 1 June 2017 (2017-06-01), pages 1 - 12, XP093198446, DOI: 10.1007/978-3-031-08337-2_4
- GEHLOT ANITA, PRAVEEN KUMAR MALIK , RAJESH SINGH , SHAIK VASEEM AKRAM , TURKI ALSUWIAN: "Dairy 4.0: Intelligent Communication Ecosystem for the Cattle Animal Welfare with Blockchain and IoT Enabled Technologies", APPLIED SCIENCES, vol. 12, no. 14, 1 January 2022 (2022-01-01), pages 1 - 19, XP093198455, ISSN: 2076-3417, DOI: 10.3390/app12147316

## Description

### TECHNICAL FIELD

The invention relates to an in-tank raw milk monitoring system and method used in the agriculture and animal husbandry sector, in the field of food production, especially in the cooling and storage tanks in raw milk collection centers, which allows remote and digital measurement of milk quality determining values such as temperature and pH and milk quantity, storage and transmission of these measurement values to remote computers, remote and real-time 24/7 monitoring of tanks, and all these operations to be carried out with minimal need for personnel intervention, which is compatible with industrial computer systems and cloud systems, sends mobile sms and e-mail notifications, can be managed by artificial intelligence thanks to the software it contains, creates a suitable ground for data mining and reporting, and saves labor, energy and time accordingly.

### PRIOR ART

One of the most important criteria determining the quality of raw milk is the pH value of the milk, which should be in a sensitive range of 6.5 to 6.7. The staff of milk producer companies go to milk collection centers, measure the pH value of milk in milk storage and cooling tanks with handheld devices, logistics production planning of milk is made according to these values, and milk of insufficient quality is not purchased and not shipped to the factory. These current methods have many disadvantages. Namely,
- pH data of milk cannot be measured digitally,
- There are between 3000 and 5000 milk collection centers all over Turkey and it is not possible to send personnel to each center and make quality control measurements,
- Since the quality controls of raw milk cannot be carried out on site, that is, in collection centers, these control procedures can be carried out after the milk is shipped to the factory and before the milk enters into production process, and raw milk that does not meet quality standards is not accepted by the factory and is disposed of,
- As a result of this process, farmers and producers suffer financial losses, and ecological damage occurs with inefficient animal husbandry, inefficient logistics and increased carbon footprint.

Although the raw milk cooling and storage tanks currently in use have the ability to digitally measure the quantity and temperature of milk, these tanks cannot measure the pH value, which is one of the most important criteria affecting milk quality. For this reason, the personnel going to milk collection centers should perform this process with handheld terminals.

There are some studies for raw milk quality and control analysis and a national patent application no. TR2022/000199 mentions a study on "Milk analysis, collection and processing system". Described herein is a milk analysis, collection and processing system that enables the analysis of the nutritional values of the milk, keeping records of the analysis values obtained, collecting statistical and financial data with the recorded data, and instant monitoring of the status of the collected milk at the moment of receipt of the milk from the producer during the raw milk collection process in dairy and/or milk and dairy products production sectors. When the above-mentioned patent document is examined, it is seen that the milk quality is determined by the personnel going to the milk collection centers using handheld devices and the data obtained are entered into the system by the personnel using handheld terminals. The document does not mention any structure that minimizes personnel intervention, performs automatic monitoring and measurement remotely and in real time, and automatically stores and shares data with remote centers. This shows that the system described in the relevant document cannot solve the problems mentioned above.

Another in-tank raw milk monitoring system is known from US 2021/176950 A1.

Thus, the need to eliminate such shortcomings and disadvantages of the embodiments and practices employed in the prior art entails an improvement in the respective technical field.

### DESCRIPTION OF THE INVENTION

The present disclosure relates to an in-tank raw milk monitoring system and method developed for eliminating the aforementioned disadvantages and providing new advantages to the respective technical field.

The aim of the invention is to provide an embodiment designed to be used in the agriculture and animal husbandry sector, in the field of food production, especially in the cooling tanks located in raw milk collection centers, allowing remote and digital measurement of milk quality determining values such as temperature and pH and milk quantity, storage and transmission of these measurement values to remote centers, remote and real-time 24/7 monitoring of the tanks and all these operations to be carried out with minimal need for personnel intervention. Another aim of the invention is to provide an embodiment that is compatible with industrial computer systems and cloud systems, sends mobile sms and e-mail notifications, and provides a suitable basis for data mining and reporting. Another aim of the invention is to provide an embodiment that saves labor, energy and time, prevents financial losses of farmers and producers, makes it possible for animal husbandry and logistics activities to achieve an efficient quality, and minimizes ecological damage by reducing carbon footprint.

The structural and characteristic features of the present disclosure, including all of its advantages, will be more clearly understood when the detailed description given below is read, and thus, the present disclosure should be evaluated by taking this detailed description into consideration.

### DETAILED DESCRIPTION OF THE INVENTION

The invention relates to an in-tank raw milk monitoring system and method used in the agriculture and animal husbandry sector, in the field of food production, especially in the cooling and storage tanks in raw milk collection centers, which allows remote and digital measurement of milk quality determining values such as temperature and pH and milk quantity, storage and transmission of these measurement values to remote computers, remote and real-time 24/7 monitoring of tanks, and all these operations to be carried out with minimal need for personnel intervention, which is compatible with industrial computer systems and cloud systems, sends mobile sms and e-mail notifications, can be managed by artificial intelligence thanks to the software it contains, creates a suitable ground for data mining and reporting, and saves labor, energy and time accordingly.

The invention comprises a system comprising hardware mounted on a raw milk cooling and storage tank and a software embedded in the hardware, and a method of operation of the system.

There are two requirements for measuring the pH value. One is to keep the pH sensor bar in a protective solution and the other is to calibrate the probe before measurements. The invention enables these two actions to be performed automatically, minimizing human intervention. With the sensors in the box mounted on the tank in which the milk is stored, all processes of the tank can be informed and the tank can be monitored remotely. Depending on the time when pH measurement is required, the pH probe automatically contacts the milk or the probe is kept in a protective solution.

The inventive system is generally composed of 12V transformer to create a direct current source for different units, Lora terminal, LTE and Lora antenna to provide wireless long distance data exchange, liquid level sensor to measure the amount of liquid coming into the sample container, pressure sensor to measure the liquid pressure in the tank, peristaltic pump to provide liquid flows, manual valve for solution filling and draining, LCD monitor for on-screen monitoring and management, pH sensor for pH determination of milk, LTE modem for mobile network connectivity, first microcomputer for data processing and management, second microcomputer for data acquisition and sensor management, Lora Gateway to collect wireless and long-distance sensor data, voltage regulator to supply different voltage values to components, temperature sensor to measure tank temperature, relay board and relay driver board to manage valves and pumps, solenoid valve to connect the sample container to the tank, the solution box for sample and solution discharge, UART card for communication between the microcomputer and the relay driver card, float to measure the full and empty status of the solution containers, UPS to provide uninterrupted power to the system, sensor circuit to perform sensor data calculation and adjustment operations.

The inventive system operates as described below.

### In general:

The first microcomputer connects to the cloud via the mobile network provided by the LTE modem and antenna and exchanges data, sending work orders to the second microcomputer or requesting measurement or status data from the second microcomputer based on the received data.

The second microcomputer requests relay status data from the relay driver board via the serial communication protocol provided by the uart board or gives relay on/off orders.

The relay driver board executes orders to open and close peristaltic pumps and valves.

The pressure and temperature of the tank are continuously measured by a pressure and temperature sensor. The sampling period can be parametrically defined for each tank. (Example 15 minutes)

The second microcomputer receives the filling amount of the sample container, temperature data of the liquid in the tank and sample container, pH value of the liquid in the sample container, pressure data of the tank, calibration data of the pH sensor, float status data (full/empty data of the liquid containers). The sensor circuit receives temperature and pH data of the solution in the sample container and temperature data of the liquid in the tank. Other sensor data is calculated by the second microcomputer conversion method.

Sensor data is sent to the first microcomputer address in the Lora network formed by the Lora terminal and antenna.

The first microcomputer receives and analyzes data coming from the Lora terminal to its own address in the Lora network provided by the Lora Gateway and antenna.

### Pressure Measurement:

The pressure sensor reports the pressure in the tank to the second microcomputer, which transfers the information to the first microcomputer that processes it and sends it to the cloud. Then check whether the drain container is full.

### Calculation of the Amount of Liquid in the Tank:

When the tank is full and empty, the amount of liquid in the tank is calculated with a calibrated pressure sensor. The second microcomputer converts the liquid pressure into liquid level and then into volume information, which is reported to the first microcomputer and then processed and sent to the cloud. If there is no liquid in the tank, the system is put into standby mode until the situation changes.

### Drain Container Process:

The float informs the second microcomputer if the drain container is full or empty, and then the information is transmitted to the first microcomputer. The first microcomputer processes it and sends it to the cloud. If the drain container is full, the full information is sent to the cloud. The system is put into standby mode until the drain container is empty.

Manual draining process is started by opening the manual valve of the drain container. Since the draining process starts with the actuation of the peristaltic pump, the system is put into standby mode until the process is finished. When the drain container is empty, the draining process of the drain container is completed with the empty information coming from the drain float with the stop of the peristaltic pump. The manual valve is closed.

### Solution Container Process:

Check that the solution containers are full together with the drain container. The floats in the solution containers inform the second microcomputer that the containers are empty, which then transfers the information to the first microcomputer. The first microcomputer processes the information and sends it to the cloud. **If** the solution containers are empty, the system is put into standby mode until the containers are full.

Solution container filling is started with manual solution filling by opening the manual valve. Since the filling process starts with the actuation of the peristaltic pump, the system is put into standby mode until the process is finished. When the solution container is full, the filling process is completed with the full information from the filling float. The manual valve is closed. **If** the solution containers are not empty, the pressure in the tank is measured.

### Tank Standby Process:

When the tank is empty, the system is in standby mode and the sampling valve is closed. The pressure and temperature of the tank are continuously measured, waiting for the start of a process that will change the system state.

### Tank Cleaning Process:

If the tank pressure value is not within the empty state pressure range of the tank, the tank temperature is controlled by the temperature sensor. If the tank pressure value is within the pressure range of the tank cleaning status and the tank temperature is within the higher tank cleaning range, the tank cleaning process starts.

The amount of liquid in the sample container is checked. If there is liquid in the sample container, the liquid is transferred to the drain container by a peristaltic pump. If there is no liquid in the sample container or if the liquid has been drained, tank cleaning solution is taken from the tank to the sample container by a peristaltic pump. After cleaning the sample container, the cleaning solution is transferred to the drain container by a peristaltic pump. After the tank cleaning, the system is allowed to stand until the next measurement time.

### Tank Filling Process:

Measurement is made when the tank pressure goes beyond the full value range, the tank temperature is within the measurement value range and liquid can be drawn from the tank. Calibration values of the sensors are checked. If there is a deterioration in the calibration, the calibration process is started. If there is no deterioration, it is proceeded with sampling from the tank.

### Calibration Process:

pH solution at pH 7.0 is transferred from the pH solution container to the sample container by a peristaltic pump. After the sensors have stable readings, calibration for pH 7.0 is performed. The calibration solution is then transferred to the drain container by a peristaltic pump. A peristaltic pump transfers the cleaning solution from the cleaning container into the sample container. The cleaning solution from the cleaned sample container is then transferred to the drain container by a peristaltic pump. After the cleaning process, pH solution at pH 4.0 is transferred from the pH solution container to the sample container by a peristaltic pump. After the sensors have stable readings, calibration for pH 4.0 is performed. The calibration solution is then transferred to the drain container by a peristaltic pump. A peristaltic pump transfers the cleaning solution from the cleaning container into the sample container. The cleaning solution from the cleaned sample container is transferred to the drain container by a peristaltic pump. Then, it is proceeded with sampling from the tank.

### Sample Measurement Process:

The sampling valve, which provides the connection between the tank and the measurement system, is only opened during the sampling process and is always closed in other cases. The measuring system is thus isolated from the tank. Sample is taken from the tank to the sample container by a peristaltic pump. The sampling valve is closed. Sample measurements are performed. The measured pH and temperature data are transferred from the second microcomputer to the first microcomputer via the Lora network. The first microcomputer processes the data it receives from the Lora network and sends it to the cloud via the mobile network. After the measurement process is finished, the system is allowed to stand until the next measurement time. (Example 15 minutes)

The first measurement is taken 2 to 3 minutes after the fluid level rises. Periodic measurement is then carried out.

### Tank Draining Process:

Draining of the tank starts with opening the tank drain valve. The tank drain float informs the second microcomputer that flow has occurred and the system is put into drain mode. Information is then transferred to the first microcomputer. The first microcomputer processes the information and sends it to the cloud. The system is taken out of drain mode when it is informed that the flow is cut off from the drain float and this information is sent to the cloud.

## Claims

1. An in-tank raw milk monitoring system that includes 12V transformer to create a direct current source for different units, a Long-Range terminal, a mobile network and a long range antenna to provide wireless long distance data exchange, liquid level sensor to measure the amount of liquid coming into the sample container, pressure sensor to measure the liquid pressure in the tank, peristaltic pump to provide liquid flows from the tank to the sample container, for draining from a drain container and filling to a solution container, manual valve for solution filling the solution container and draining the drain container, a liquid-crystal display (LCD) monitor for on-screen monitoring and management, pH sensor for pH determination of milk, mobile network modem for mobile network connectivity, first microcomputer for data processing and management, second microcomputer for data acquisition and sensor management, a long range Gateway to collect wireless and long-distance sensor data, voltage regulator to supply different voltage values to components, temperature sensor to measure tank temperature, relay board and relay driver board to manage valves and pumps, solenoid valve to connect the sample container to the tank, the solution box for sample and solution discharge, a universal asynchronous receiver/transmitter (UART) card for communication between the microcomputer and the relay driver card, float to measure the full and empty status of the solution containers, an uninterruptible power supply (UPS) to provide uninterrupted power to the system, sensor circuit to perform sensor data calculation and adjustment operations.

2. The in-tank raw milk monitoring method comprising the process steps of
- in general, connecting to the cloud and exchanging data by the first microcomputer with the mobile network provided by the mobile network modem and antenna, sending work orders to the second microcomputer or requesting measurement or status data from the second microcomputer based on the received data, requesting relay status data by the second microcomputer from the relay driver board via the serial communication protocol provided by the universal asynchronous receiver/transmitter (UART) board, or issuing relay on/off orders, executing the orders to open and close the peristaltic pumps and valves by the relay driver board, measuring the pressure and temperature of the tank continuously by a pressure and temperature sensor and parametrically defining the sampling period for each tank, receiving data on the fullness of the sample container, temperature data of the liquid in the tank and sample container, pH value of the liquid in the sample container, pressure data of the tank, calibration data of the pH sensor, float status data by the second microcomputer, receiving the temperature and pH data of the solution in the sample container and the temperature data of the liquid in the tank by the sensor circuit, calculating the other sensor data by the second microcomputer through the conversion method, and sending the sensor data to the first microcomputer address in the long range network formed by the long range terminal and long range antenna, receiving and analyzing data by the first microcomputer from the long range terminal to its own address in the long range network provided by the long range Gateway and long range antenna,
- as a part of the pressure measurement process, reporting the pressure in the tank by the pressure sensor to the second microcomputer, then transferring information to the first microcomputer, processing it and sending the information to the cloud, then checking whether the drain container is full or not,
- as a part of the calculating the amount of liquid in the tank, calculating the amount of liquid in the tank with a calibrated pressure sensor when the tank is full and empty, converting the liquid pressure into liquid level and then into volume information by the second microcomputer and reporting this information to the first microcomputer, then processing the information and sending it to the cloud, if there is no liquid in the tank, putting the system into standby mode until the situation changes,
- as a part of the drain container process, informing the second microcomputer by the float when the drain container is full or empty, and then transferring information to the first microcomputer, processing the information and sending it to the cloud by the first microcomputer, sending full information to the cloud if the drain container is full, putting the system in standby mode until the drain container is empty, starting manual draining process by opening the manual valve of the drain container, since the draining process starts with the actuation of the peristaltic pump, putting the system into standby mode until the process is finished, when the drain container is empty, stopping the peristaltic pump and draining the drain container based on the empty information from the drain float and closing the manual valve,
- as a part of the solution container process, checking that the solution containers are full together with the drain container, informing the second microcomputer that the containers are empty by the floats in the solution containers, then transferring the information to the first microcomputer, processing the information and sending it to the cloud by the first microcomputer, if the solution containers are empty, putting the system into standby mode until the containers are filled, starting solution container filling with the opening of the manual valve, since the filling process starts with the actuation of the peristaltic pump, putting the system into standby mode until the process is finished, when the solution container is full, completing the filling process with the full information from the filling float and closing the manual valve, if the solution containers are not empty, starting the pressure measurement in the tank,
- as a part of the tank standby process, keeping the system in standby mode and the sample valve in the closed position when the tank is empty, continuously measuring the pressure and temperature of the tank and waiting for the start of a process that will change the system state,
- as a part of the tank cleaning process, checking the tank temperature with the temperature sensor if the tank pressure value is not in the empty state pressure range of the tank, starting the tank cleaning process if the tank pressure value is in the cleaning state pressure range of the tank and the tank temperature is in the higher tank cleaning range, checking the amount of liquid in the sample container, if there is liquid in the sample container, transferring the liquid to the drain container by a peristaltic pump, if there is no liquid in the sample container or if the liquid has been drained, taking the tank cleaning solution from the tank to the sample container by a peristaltic pump, after the sample container is cleaned, transferring the cleaning solution to the drain container by a peristaltic pump, after the tank cleaning is finished, allowing the system to stand until the next measurement time,
- as a part of the tank filling process, measuring if the tank pressure goes beyond to the full value range, the tank temperature is within the measurement value range and liquid can be drawn from the tank, checking the calibration values of the sensors, starting the calibration process if there is a deterioration in the calibration, and if there is no deterioration, proceed with the sampling process from the tank
- as a part of the calibration process, transferring the solution at pH 7.0 from the pH 7.0 solution container to the sample container by a peristaltic pump, performing calibration for pH 7.0 after the sensors make a stable reading, then transferring the calibration solution to the drain container with a peristaltic pump, transferring the cleaning solution from the cleaning container to the sample container by a peristaltic pump, then transferring the cleaning solution in the cleaned sample container to the drain container by a peristaltic pump, transferring the pH solution from the pH 4.0 solution container to the sample container by a peristaltic pump after cleaning, performing pH 4.0 calibration after the sensors have stable readings, then transferring the calibration solution to the drain container by a peristaltic pump, transferring the cleaning solution from the cleaning container to the sample container by a peristaltic pump, transferring the cleaning solution in the cleaned sample container to the drain container by a peristaltic pump, then proceeding to the sampling process from the tank,
- as a part of the sample measurement process, opening the sample valve connecting the tank to the measurement system only during the sampling process and always closing it in other cases, taking samples from the tank to the sample container by a peristaltic pump, closing the sample valve and taking sample measurements, taking the first measurement 2 to 3 minutes after the liquid level rises, then making the periodic measurements, transferring the measured pH and temperature data from the second microcomputer to the first microcomputer via the long range network, processing the data received from the long range network and sending it to the cloud via the mobile network by the first microcomputer, after the measurement process is over, allowing the system to stand until the next measurement time,
- as a part of the tank draining process, starting the drainage of the tank with the opening of the tank drain valve, informing the second microcomputer by the tank drain float that flow has occurred and putting the system into drain mode, then tranferring information to the first microcomputer, processing the information and sending it to the cloud by the first microcomputer, removing the system from the drain mode when the information that the flow has stopped is received from the drain float and sending this information to the cloud.

## Patentansprüche

1. System zur Überwachung von Rohmilch im Tank, das einen 12-V-Transformator zur Erzeugung einer Gleichstromquelle für verschiedene Einheiten, ein Long-Range-Terminal, ein Mobilfunknetz sowie eine Long-Range-Antenne für den drahtlosen Datenaustausch über große Entfernungen einschließt, einen Füllstandssensor zur Messung der in den Probenbehälter einströmenden Flüssigkeitsmenge, einen Drucksensor zur Messung des Flüssigkeitsdrucks im Tank, eine Peristaltikpumpe, um Flüssigkeit aus dem Tank in den Probenbehälter zu stellen, zum Entleeren eines Ablaufbehälters und zum Befüllen eines Lösungsbehälters, ein manuelles Ventil zum Befüllen des Lösungsbehälters und zum Entleeren des Ablaufbehälters, einen LCD-Monitor zur Überwachung und Verwaltung auf dem Bildschirm, einen pH-Sensor zur Bestimmung des pH-Werts von Milch, ein Mobilfunkmodem für die Anbindung an das Mobilfunknetz, einen ersten Mikrocomputer zur Datenverarbeitung und -verwaltung, einen zweiten Mikrocomputer zur Datenerfassung und Sensorverwaltung, ein Long-Range-Gateway zur Erfassung drahtloser und über großen Abstand übertragener Sensordaten, einen Spannungsregler zur Versorgung der Komponenten mit unterschiedlichen Spannungswerten, einen Temperatursensor zur Messung der Tanktemperatur, eine Relaisplatine und eine Relais-Treiberplatine zur Steuerung von Ventilen und Pumpen, ein Magnetventil zum Verbinden des Probenbehälters mit dem Tank, die Lösungsbehälter zum Abgeben von Proben und Lösungen, eine UART-Karte (Universal Asynchronous Receiver/Transmitter) zur Kommunikation zwischen dem Mikrocomputer und der Relais-Treiberplatine, einen Schwimmer zur Erfassung des Füll- und Leerstands der Lösungsbehälter, eine unterbrechungsfreie Leistungsversorgung (USV) zur unterbrechungsfreien Leistungsversorgung des Systems sowie eine Sensorschaltung zur Durchführung von Berechnungen und Einstellvorgängen der Sensordaten.

2. Verfahren zur Überwachung von Rohmilch im Tank, umfassend folgende Verfahrensschritte:
- im Allgemeinen, Verbinden mit der Cloud und Austauschen von Daten durch den ersten Mikrocomputer über das Mobilfunknetz, das durch das Mobilfunkmodem und die Antenne bereitgestellt wird; Senden von Arbeitsaufträgen an den zweiten Mikrocomputer oder Anfordern von Mess- oder Statusdaten vom zweiten Mikrocomputer basierend auf den empfangenen Daten; Anforderung von Relais-Statusdaten durch den zweiten Mikrocomputer von der Relais-Treiberplatine über das serielle Kommunikationsprotokoll, das von der Universal Asynchronous Receiver/Transmitter (UART)-Karte, oder das Erteilen von Befehlen zum Ein- und Ausschalten der Relais, das Ausführen der Befehle zur Öffnung und zum Verschließen der Peristaltikpumpen und Ventile durch die Relais-Treiberplatine, das kontinuierliche Messen von Druck und Temperatur des Tanks mittels eines Druck- und Temperatursensors sowie die parametrische Festlegung der Abtastperiode für jeden Tank, das Empfangen von Daten zum Füllstand des Probenbehälters, von Temperaturdaten der Flüssigkeit im Tank und im Probenbehälter, den pH-Wert der Flüssigkeit im Probenbehälter, die Druckdaten des Tanks, die Kalibrierungsdaten des pH-Sensors sowie die Schwimmerzustandsdaten durch den zweiten Mikrocomputer; das Empfangen der Temperatur- und pH-Daten der Lösung im Probenbehälter sowie der Temperaturdaten der Flüssigkeit im Tank durch die Sensorschaltung; das Berechnen der übrigen Sensordaten durch den zweiten Mikrocomputer mittels eines Umwandlung-Verfahrens; sowie Senden der Sensordaten an die Adresse des ersten Mikrocomputers in dem durch das Fernbereichsterminal und die Fernbereichsantenne gebildeten Fernbereichsnetzwerk; Empfangen und Analysieren von Daten durch den ersten Mikrocomputer, die vom Fernbereichsterminal an seine eigene Adresse im Fernbereichsnetzwerk gesendet werden, das durch das Fernbereichs-Gateway und die Fernbereichsantenne bereitgestellt wird,
- im Rahmen des Druckmessvorgangs, Melden des Drucks im Tank im Rahmen des Druckmessvorgangs, Melden des Drucks im Tank durch den Drucksensor an den zweiten Mikrocomputer, Übertragung der Informationen an den ersten Mikrocomputer, Verarbeiten dieser Informationen und Weiterleiten an die Cloud, anschließend Überprüfen, ob der Ablaufbehälter voll ist oder nicht,
- im Rahmen der Berechnung der Flüssigkeitsmenge im Tank, Berechnen der Flüssigkeitsmenge im Tank mit einem kalibrierten Drucksensor, wenn der Tank voll oder leer ist, Umwandeln des Flüssigkeitsdrucks in einen Level und anschließend in Volumenangaben durch den zweiten Mikrocomputer sowie Meldung dieser Informationen an den ersten Mikrocomputer, anschließendes Verarbeiten der Informationen und deren Übermitteln an die Cloud; falls sich keine Flüssigkeit im Tank befindet, Versetzung des Systems in den Standby-Modus, bis es zu einer Veränderung der Situation kommt,
- im Rahmen des Ablaufbehältervorgangs, Übermitteln der Informationen an den zweiten Mikrocomputer durch den Schwimmer, sobald der Ablaufbehälter voll oder leer ist, und anschließendes Weiterleiten der Informationen an den ersten Mikrocomputer, Verarbeiten der Informationen und Senden an die Cloud durch den ersten Mikrocomputer, Senden einer "voll"-Meldung an die Cloud, falls der Ablaufbehälter voll ist, Versetzen des Systems in den Standby-Modus, bis der Ablaufbehälter leer ist, Starten des manuellen Entleerungsvorgangs durch Öffnung des Handventils des Ablaufbehälters, da der Entleerungsvorgang mit der Aktivierung der Peristaltikpumpe beginnt, Versetzen des Systems in den Standby-Modus, bis der Vorgang abgeschlossen ist; wenn der Ablaufbehälter leer ist, die Peristaltikpumpe beenden und Entleeren des Ablaufbehälters basierend auf den Informationen des Schwimmers sowie das Handventil verschließen,
- im Rahmen des Lösungsbehältervorgangs, Überprüfen, ob die Lösungsbehälter zusammen mit dem Ablaufbehälter voll sind, Informieren des zweiten Mikrocomputers über den leeren Zustand der Behälter mittels der Schwimmer in den Lösungsbehältern, anschließendes Weiterleiten der Informationen an den ersten Mikrocomputer, Verarbeiten der Informationen und Senden an die Cloud durch den ersten Mikrocomputer; falls die Lösungsbehälter leer sind, Versetzen des Systems in den Standby-Modus, bis die Behälter gefüllt sind, Starten des Befüllvorgangs der Lösungsbehälter durch Öffnung der Öffnung des Handventils, da der Befüllvorgang mit der Betätigung der Peristaltikpumpe beginnt, Versetzen des Systems in den Standby-Modus, bis der Vorgang abgeschlossen ist; wenn der Lösungsbehälter voll ist, Abschließen des Befüllvorgangs anhand der Füllstandsmeldung des Schwimmers und Verschließen des Handventils; falls die Lösungsbehälter nicht leer sind, Starten der Druckmessung im Tank,
- im Rahmen des Tank-Standby-Prozesses, halten des Systems im Standby-Modus und das Probenventil in verschlossener Position, wenn der Tank leer ist, kontinuierliches Messen des Drucks und der Temperatur des Tanks sowie Warten auf den Start eines Prozesses, der eine Veränderung des Systemzustands bewirkt,
- im Rahmen des Tankreinigungsvorgangs, Überprüfen der Tanktemperatur mit dem Temperatursensor, falls der Tankdruckwert nicht im Druckbereich des leeren Zustands des Tanks liegt; Starten des Tankreinigungsvorgangs, falls der Tankdruckwert im Druckbereich des Reinigungszustands des Tanks liegt und die Tanktemperatur im höheren Tankreinigungsbereich liegt; Überprüfen der Flüssigkeitsmenge im Probenbehälter; falls sich Flüssigkeit im Probenbehälter befindet, Übertragen der Flüssigkeit mittels einer Peristaltikpumpe in den Ablassbehälter; falls der Probenbehälter keine Flüssigkeit aufweist oder die Flüssigkeit bereits abgelassen wurde, Befüllen des Probenbehälters mit der Tankreinigungslösung aus dem Tank mittels einer Peristaltikpumpe; nach der Reinigung des Probenbehälters Übertragen der Reinigungslösung mittels einer Peristaltikpumpe in den Ablaufbehälter; nach Abschluss der Tankreinigung das System bis zum nächsten Messzeitpunkt ruhen lassen,
- im Rahmen des Tankbefüllvorgangs, Messung, ob der Tankdruck den oberen Grenzwert des Messbereichs überschreitet, ob die Tanktemperatur innerhalb des Messbereichs liegt und ob Flüssigkeit aus dem Tank entnommen werden kann; Überprüfen der Kalibrierwerte der Sensoren; Starten des Kalibriervorgangs bei einer Verschlechterung der Kalibrierung; und, falls keine Verschlechterung vorliegt, Fortsetzen des Probenahmevorgangs aus dem Tank
- im Rahmen des Kalibriervorgangs, Übertragen der Lösung mit einem pH-Wert von 7,0 aus dem pH-7,0-Behälter in den Probenbehälter mittels einer Schlauchpumpe, Durchführen der Kalibrierung für pH 7,0, nachdem die Sensoren einen stabilen Messwert anzeigen, anschließend Übertragen der Kalibrierlösung in den Abflussbehälter mit einer Schlauchpumpe, Übertragen der Reinigungslösung aus dem Reinigungsbehälter in den Probenbehälter mittels einer Peristaltikpumpe, anschließend Übertragen der Reinigungslösung aus dem gereinigten Probenbehälter in den Abflussbehälter mittels einer Peristaltikpumpe, Übertragen der pH-Lösung aus dem pH-4,0-Lösungsbehälter in den Probenbehälter mittels einer Peristaltikpumpe nach der Reinigung, Durchführen der pH-4,0-Kalibrierung, nachdem die Sensoren stabile Messwerte anzeigen, sowie das anschließende Übertragen der Kalibrierlösung in den Abflussbehälter mittels einer Peristaltikpumpe, Übertragen der Reinigungslösung aus dem Reinigungsbehälter in den Probenbehälter mittels einer Peristaltikpumpe, Übertragen der Reinigungslösung aus dem gereinigten Probenbehälter in den Abflussbehälter mittels einer Peristaltikpumpe, anschließend Fortsetzen des Probenahmevorgangs aus dem Tank,
- im Rahmen des Probenmessvorgangs, Öffnen des Probenventils, das den Tank mit dem Messsystem verbindet, ausschließlich während des Probenahmevorgangs und stets sein Verschließen in allen anderen Fällen, Entnahme von Proben aus dem Tank in den Probenbehälter mittels einer Peristaltikpumpe, Verschließen des Probenventils und Durchführen von Probenmessungen, Durchführen der ersten Messung 2 bis 3 Minuten nach dem Anstieg des Flüssigkeitsstands, anschließend Durchführen der periodischen Messungen, Übertragen der gemessenen pH- und Temperaturdaten vom zweiten Mikrocomputer zum ersten Mikrocomputer über das Fernnetzwerk; Verarbeiten der vom Fernnetzwerk empfangenen Daten und deren Übermitteln an die Cloud über das Mobilfunknetz durch den ersten Mikrocomputer; nach Beendigung des Messvorgangs, Ruhenlassen des Systems bis zum nächsten Messzeitpunkt,
- im Rahmen des Tankentleerungsvorgangs, Starten der Entleerung des Tanks mit der Öffnung des Tankablassventils, Übermitteln der Informationen an den ersten Mikrocomputer durch den Tankentleerungsschwimmer, der den zweiten Mikrocomputer darüber informiert, dass ein Durchfluss stattgefunden hat, und Versetzen des Systems in den Entleerungsmodus; anschließendes Verarbeiten der Informationen durch den ersten Mikrocomputer und Senden dieser an die Cloud; Beenden des Entleerungsmodus, sobald die Informationen über das Beenden des Durchflusses vom Entleerungsschwimmer empfangen werden, und Senden dieser Informationen an die Cloud.

## Revendications

1. Système de surveillance de lait cru dans un réservoir qui comporte un transformateur 12 V pour créer une source de courant continu pour des différentes unités, un terminal Long Range, un réseau mobile et une antenne Long Range pour assurer un échange de données à longue distance sans fil, un capteur de niveau de liquide pour mesurer la quantité de liquide entrant dans le récipient d'échantillon, un capteur de pression pour mesurer la pression de liquide dans le réservoir, une pompe péristaltique pour assurer des flux de liquide à partir du réservoir jusqu'au récipient d'échantillon, pour le drainage à partir d'un récipient de drainage et pour le remplissage d'un récipient de solution, une vanne manuelle pour le remplissage du récipient de solution et le drainage du récipient de drainage, un afficheur à cristaux liquides (LCD) pour la surveillance et la gestion sur écran, un capteur de pH pour la détermination de pH du lait, un modem de réseau mobile pour la connectivité au réseau mobile, un premier micro-ordinateur pour le traitement et la gestion de données, un deuxième micro-ordinateur pour l'acquisition de données et la gestion de capteurs, une passerelle Long Range pour collecter des données de capteur sans fil et à longue distance, un régulateur de tension pour fournir différentes valeurs de tension à des composants, un capteur de température pour mesurer la température de réservoir, la carte relais et la carte de pilote de relais pour gérer des vannes et des pompes, une électrovanne pour relier le récipient d'échantillon au réservoir, la boîte de solution pour une décharge d'échantillon et de solution, une carte émetteur/récepteur asynchrone universel (UART) pour la communication entre le micro-ordinateur et la carte de pilote de relais, un flotteur pour mesurer l'état plein et vide des récipients de solution, une alimentation sans interruption (UPS) pour assurer une puissance sans interruption au système, un circuit de capteur pour effectuer des opérations de calcul et d'ajustement de données de capteur.

2. Procédé de surveillance de lait cru dans un réservoir comprenant les étapes de processus suivantes :
- en général, la connexion au cloud et l'échange de données par le premier micro-ordinateur avec le réseau mobile assuré par le modem de réseau mobile et l'antenne, l'envoi de commandes de travail vers le deuxième micro-ordinateur ou la demande de données de mesure ou d'état à partir du deuxième micro-ordinateur sur la base des données reçues, la demande de données d'état de relais par le deuxième micro-ordinateur à partir de la carte de pilote de relais via le protocole de communication série fourni par la carte émetteur/récepteur asynchrone universel (UART), ou l'émission de commandes de marche/arrêt de relais, l'exécution des commandes pour ouvrir et fermer les pompes péristaltiques et les vannes par la carte de pilote de relais, la mesure de la pression et de la température du réservoir en continu par un capteur de pression et de température et la définition paramétrique de la période d'échantillonnage pour chaque réservoir, la réception de données sur le remplissage du récipient d'échantillon, de données de température du liquide dans le réservoir et le récipient d'échantillon, d'une valeur de pH du liquide dans le récipient d'échantillon, de données de pression du réservoir, de données d'étalonnage du capteur de pH, de données d'état de flotteur par le deuxième micro-ordinateur, la réception des données de température et de pH de la solution dans le récipient d'échantillon et des données de température du liquide dans le réservoir par le circuit de capteur, le calcul des autres données de capteur par le deuxième micro-ordinateur à travers le procédé de conversion, et l'envoi des données de capteur vers la première adresse de micro-ordinateur dans le réseau Long Range formé par le terminal Long Range et l'antenne Long Range, la réception et l'analyse de données par le premier micro-ordinateur en provenance du terminal Long Range vers sa propre adresse dans le réseau Long Range fourni par la passerelle Long Range et l'antenne Long Range,
- comme partie du processus de mesure de pression, la transmission de la pression dans le réservoir par le capteur de pression au deuxième micro-ordinateur, puis le transfert des informations vers le premier micro-ordinateur, le traitement et l'envoi des informations vers le cloud, puis la vérification du remplissage plein, ou non, du récipient de drainage,
- comme partie du calcul de la quantité de liquide dans le réservoir, le calcul de la quantité de liquide dans le réservoir avec un capteur de pression étalonné lorsque le réservoir est plein et vide, la conversion de la pression de liquide en niveau de liquide puis en informations de volume par le deuxième micro-ordinateur et la transmission de ces informations au premier micro-ordinateur, puis le traitement des informations et l'envoi de celles-ci vers le cloud, s'il n'y a pas de liquide dans le réservoir, la mise du système en mode veille jusqu'à ce que la situation change,
- comme partie du processus de récipient de drainage, la notification du deuxième micro-ordinateur par le flotteur lorsque le récipient de drainage est plein ou vide, puis le transfert des informations vers le premier micro-ordinateur, le traitement des informations et l'envoi de celles-ci vers le cloud par le premier micro-ordinateur, l'envoi de l'information plein vers le cloud si le récipient de drainage est plein, la mise du système en mode veille jusqu'à ce que le récipient de drainage soit vide, le démarrage d'un processus de drainage manuel en ouvrant la vanne manuelle du récipient de drainage, puisque le processus de drainage débute avec l'actionnement de la pompe péristaltique, la mise du système en mode veille jusqu'à ce que le processus soit terminé, lorsque le récipient de drainage est vide, l'arrêt de la pompe péristaltique et le drainage du récipient de drainage sur la base de l'information vide en provenance du flotteur de drain et la fermeture de la vanne manuelle,
- comme partie du processus de récipient de solution, la vérification que les récipients de solution ainsi que le récipient de drainage sont pleins, la notification du deuxième micro-ordinateur que les récipients sont vides par les flotteurs dans les récipients de solution, puis le transfert des informations vers le premier micro-ordinateur, le traitement des informations et l'envoi de celles-ci vers le cloud par le premier micro-ordinateur, si les récipients de solution sont vides, la mise du système en mode veille jusqu'à ce que les récipients soient remplis, le démarrage du remplissage de récipient de solution avec l'ouverture de la vanne manuelle, puisque le processus de remplissage débute avec l'actionnement de la pompe péristaltique, la mise du système en mode veille jusqu'à ce que le processus soit terminé, lorsque le récipient de solution est plein, la terminaison du processus de remplissage avec l'information plein en provenance du flotteur de remplissage et la fermeture de la vanne manuelle, si les récipients de solution ne sont pas vides, le démarrage de la mesure de pression dans le réservoir,
- comme partie du processus de veille de réservoir, le maintien du système en mode veille et de la vanne d'échantillon en position fermée lorsque le réservoir est vide, le mesure en continu de la pression et de la température du réservoir et l'attente du début d'un processus qui changera l'état du système,
- comme partie du processus de nettoyage de réservoir, la vérification de la température de réservoir avec le capteur de température si la valeur de pression de réservoir n'est pas dans la plage de pression d'état vide du réservoir, le démarrage du processus de nettoyage de réservoir si la valeur de pression de réservoir est dans la plage de pression d'état de nettoyage du réservoir et que la température de réservoir est dans la plage de nettoyage de réservoir supérieure, la vérification de la quantité de liquide dans le récipient d'échantillon, s'il y a du liquide dans le récipient d'échantillon, le transfert du liquide vers le récipient de drainage par une pompe péristaltique, s'il n'y a pas de liquide dans le récipient d'échantillon ou si le liquide a été drainé, la prise de la solution de nettoyage de réservoir à partir du réservoir jusqu'au récipient d'échantillon par une pompe péristaltique, après nettoyage du récipient d'échantillon, le transfert de la solution de nettoyage vers le récipient de drainage par une pompe péristaltique, à l'issue du nettoyage de réservoir, la mise au système en veille jusqu'à l'instant de mesure suivant,
- comme partie du processus de remplissage de réservoir, le détermination de la pression de réservoir allant au-delà de la plage de valeurs au plein, de la température de réservoir se trouvant dans la plage de valeurs de mesure et du liquide pouvant être prélevé du réservoir, la vérification des valeurs d'étalonnage des capteurs, le démarrage du processus d'étalonnage s'il y a une détérioration de l'étalonnage, et s'il n'y a pas de détérioration, la réalisation du processus d'échantillonnage à partir du réservoir,
- comme partie du processus d'étalonnage, le transfert de la solution au pH 7,0 à partir du récipient de solution au pH 7,0 vers le récipient d'échantillon par une pompe péristaltique, la réalisation d'un étalonnage pour un pH 7,0 une fois que les capteurs donnent une lecture stable, puis le transfert de la solution d'étalonnage vers le récipient de drainage avec une pompe péristaltique, le transfert de la solution de nettoyage à partir du récipient de nettoyage vers le récipient d'échantillon par une pompe péristaltique, puis le transfert de la solution de nettoyage dans le récipient d'échantillon nettoyé vers le récipient de drainage par une pompe péristaltique, le transfert de la solution de pH à partir du récipient de solution au pH 4,0 vers le récipient d'échantillon par une pompe péristaltique après nettoyage, la réalisation d'un étalonnage au pH 4,0 une fois que les capteurs donnent une lecture stable, puis le transfert de la solution d'étalonnage vers le récipient de drainage par une pompe péristaltique, le transfert de la solution de nettoyage à partir du récipient de nettoyage vers le récipient d'échantillon par une pompe péristaltique, le transfert de la solution de nettoyage dans le récipient d'échantillon nettoyé vers le récipient de drainage par une pompe péristaltique, puis la réalisation du processus d'échantillonnage à partir du réservoir,
- comme partie du processus de mesure d'échantillon, l'ouverture de la vanne d'échantillon reliant le réservoir au système de mesure seulement pendant le processus d'échantillonnage et toujours la fermeture de celle-ci dans les autres cas, la prise d'échantillons à partir du réservoir vers le récipient d'échantillon par une pompe péristaltique, la fermeture de la vanne d'échantillon et la prise de mesures d'échantillon, la prise de la première mesure 2 à 3 minutes après la montée du niveau de liquide, puis la réalisation des mesures périodiques, le transfert des données de pH et de température mesurées à partir du deuxième micro-ordinateur vers le premier micro-ordinateur via le réseau Long Range, le traitement des données reçues en provenance du réseau Long Range et l'envoi de celles-ci vers le cloud via le réseau mobile par le premier micro-ordinateur, à l'issue du processus de mesure, la mise du système en veille jusqu'à l'instant de mesure suivant,
- comme partie du processus de drainage de réservoir, le démarrage du drainage du réservoir avec l'ouverture de la vanne de vidange de réservoir, la notification du deuxième micro-ordinateur par le flotteur de drain de réservoir que le flux s'est produit et la mise du système en mode drainage, puis le transfert des informations vers le premier micro-ordinateur, le traitement des informations et l'envoi de celles-ci vers le cloud par le premier micro-ordinateur, la mise du système hors mode drainage lorsque les informations indiquant que le flux s'est arrêté sont reçues en provenance du flotteur de drain et l'envoi de ces informations vers le cloud.
